# EUROPEAN PATENT APPLICATION

(11) **EP 4 825 167 A1**
(43) Date of publication of application: **21.10.2026**
(21) Application number: 24903699.7
(22) Date of filing: 10.12.2024
(51) Int. Cl.: A01K 67/00, A61K 35/12, C12Q 1/02

(54) **METHOD FOR PRODUCING TRANSPLANTATION MODEL ANIMAL FOR EVALUATING TRANSPLANTATION MATERIAL**

(30) Priority: 11.12.2023 JP 2023208198
(71) Applicant: Miyauchi, Hiroshi, Tokyo 178-0061 (JP)
(72) Inventor: Miyauchi, Hiroshi, Tokyo 178-0061 (JP)
(74) Representative: Schön, Christoph
(86) International application number: PCT/JP2024/043706
(87) International publication number: WO 2025/127045

(57) **Abstract**

[Technical Problem]

To provide a method for producing a transplantation model animal capable of realizing a highly reliable and highly reproducible transplantation experimental system for designing transplant materials having performance and quality better suited to clinical medicine.

[Solution to Problem]

A method for evaluating a transplant material approximating autotransplantation according to the present invention comprises obtaining a recipient that is immunotolerant to a donor expressing a marker by administering a crude extract of the donor to a neonatal recipient, and transplanting, into the immunotolerant recipient, a transplant material including an artificial transplant scaffold coated with, adhered to, infiltrated with, or containing tissue or cells of the donor expressing a marker, thereby providing a highly reliable and highly reproducible transplantation experimental system approximating autotransplantation

## Description

### [Technical Field]

The present invention relates to a method for evaluating a transplant material, which is usable as a highly reliable and highly reproducible experimental system approximating autotransplantation and which is prepared with high homology to clinical medicine.

### [Background]

Regenerative medicine is a technology for repairing or supplementing the function and/or structure of living tissue in animals such as humans, livestock, and companion animals when the function and/or structure of the living tissue has been damaged or lost. Regenerative medicine is also useful when, during the developmental or growth stage of an animal, growth is inhibited by some disease or trauma and a function and/or structure that should have been obtained has not been obtained.

As one of the most effective means in regenerative medicine, a method is used in which a biologically derived medical material containing cells or tissue is transplanted into a defective part. In Japan, such medical materials are defined as "regenerative medicine products" under the three laws related to regenerative medicine (the Regenerative Medicine Promotion Act, the Act on the Safety of Regenerative Medicine, and the revised Pharmaceutical Affairs Act).

From ethical and immunological viewpoints, it is desirable that regenerative medicine products used for transplantation be those produced by processing materials derived from the individual receiving the transplantation, that is, those used for "autoplastic transplantation."

Artificial materials are also used in order to complement elements that are insufficient when only biologically derived medical materials are used. Such artificial materials are required not to cause undesirable reactions in vivo, including immune reactions. Conventionally, not only biologically derived materials including regenerative medicine products, but also various medical materials called biocompatible materials and bioaffinitive materials have been used (for example, Patent Documents 1 to 4). However, it is difficult to verify before transplantation whether those materials have the optimum material, physical properties, and configuration. In particular, it has been difficult to verify in advance the performance of regenerative medicine products containing living cells and tissues.

In order to ensure the quality of regenerative medicine products, it is necessary to have a clinically relevant system in which animal experiments are performed in advance using mammals and the course of events such as immune reactions after transplantation can be followed. For such animal experiments, an animal experimental model in which "autotransplantation" is performed, in which cells and tissues collected from an individual scheduled for transplantation are returned to that individual, is suitable.

However, animal experimental systems using autotransplantation have hardly been used as a research means up to now. In particular, there has not even been an autotransplantation experimental model for small animals. The reason is that autotransplantation is difficult in small animals including mice and rats. Even when the amount of cells or tissue collected for transplantation is less than 1 g, small animals may not withstand the collection procedure, or, depending on the tissue collected, the amount may be fatal. In small animals, in general, tissues and cells are collected after the individual is sacrificed and exsanguinated. Therefore, the individual is lost due to the collection procedure, and it has been impossible to perform autotransplantation.

In medium-sized animals and large animals, autotransplantation is possible, but because rearing is costly, it is difficult to secure a sufficient number of experimental cases. In addition, with the recent spread of the spirit of animal welfare, there is a trend toward strengthening regulation of animal experiments themselves.

Therefore, much of the conventional research has been constructed by experimental systems centered on allograft and heterograft/xenograft in small animals. Since allograft and heterograft/xenograft are not autotransplantation, in order to avoid immune reactions, it is necessary to use immunosuppressants or severely immunodeficient animals (such as nude mice, NOD/scid mice, and NOG mice), or a combination thereof. Therefore, it is necessary to use individuals whose production and survival maintenance are costly, and this has become a problem in that expenses and labor increase. Furthermore, in such experimental systems, because the risk of infection is high due to the use of immunosuppressants or immunodeficiency, it has been necessary to use advanced dedicated facilities at the sterile-room level. There has also been a problem in that immunosuppressants must be used for a long period, resulting in further cost.

Furthermore, a major problem is that immunodeficient experimental systems constitute experiments under conditions greatly divergent from clinical practice in medicine and veterinary medicine. Particularly in humans, patients in an immunodeficient state and patients under long-term use of immunosuppressants are almost never the subjects of transplant therapy. When treatments established in immunodeficient experimental systems are introduced into patients and livestock having normal immune function, unforeseen events in terms of immune reaction may occur. Accordingly, the present invention provides an animal experimental system that makes it possible to design and develop transplant materials having more appropriate performance and quality while avoiding these various problems. In particular, the present invention provides an animal experimental system capable of evaluating engraftment of a target tissue and reproducibility of structure/function. More specifically, the present invention provides an animal experimental system capable of evaluating not only minimization of rejection reactions but also the presence/absence and appropriateness/inappropriateness of reactions that support/assist the transplanted tissue (such as induction of blood flow/angiogenesis/expression regulation of nutritional factors).

### [Prior Art Documents]

### [Patent Documents]

[Patent Document 1] Japanese unexamined patent publication No. 2015-89433
[Patent Document 2] Japanese unexamined patent publication No. 2007-186831
[Patent Document 3] Japanese unexamined patent publication No. 2013-162796
[Patent Document 4] Japanese unexamined patent publication No. 2008-523957

### [Summary of the Invention]

### [Problems to be Solved by the Invention]

There is a demand for construction of a reliable and reproducible system for verifying whether biologically derived materials including regenerative medicine products, as well as biocompatible materials and bioaffinitive materials, which serve as materials used in transplantation, have the optimal composition and configuration for treatment. For that purpose, transplantation experiments using animals are indispensable, and transplant materials should be designed on that basis.

Considering cost and the like, it is suitable to use small animals in animal experiments. On the other hand, in small animals, because the body size is small, the individual dies as a sacrifice at the time of cell collection, and therefore autotransplantation is difficult. On the other hand, when allogeneic transplantation is performed, immune reactions after transplantation (such as immune rejection) occur, and therefore experiments are conducted in immunodeficient experimental systems and experimental systems using immunosuppressants. However, such experimental systems are greatly divergent from the actual conditions of treatment in clinical practice and lack reliability and reproducibility. In addition, immunodeficient experimental systems also have problems in that experimental materials are costly and highly specialized equipment is required.

Accordingly, an object of the present invention is to provide a method for producing a transplantation model animal capable of realizing a highly reliable and highly reproducible transplantation experimental system.

### [Means for Solving the Problems]

Medical materials for transplantation (Donor Graft) that are ordinarily used include auto-graft, allograft, and heterograft/xenograft, but all of them are Raw Grafts (unprocessed transplant materials) in which collected tissue is transplanted "soon" without particular processing. "without delay" includes not only the case where transplantation is performed immediately after collection, but also cases where a certain period of time is required for refrigeration/freezing storage or separation and purification of components, as in component transfusion, blood products, and autologous blood transfusion. These Raw Grafts are premised on being transplanted for the purpose of using the traits/state of the cells and tissues at the time of collection as they are. Because they are used as the relevant tissue itself without processing and with the traits/state as they are, it has basically been considered unnecessary to evaluate their performance/quality.

On the other hand, the medical material "Auto-plast," which is a coined term in the present invention and is used in what is referred to in the present invention as "Auto-plastic transplantation," is produced as a medical material for transplantation having traits/state completely different from the traits/state at the time of collection as a result of various processings such as differentiation and transdifferentiation in accordance with the cells or tissues to be transplanted, is a material not included in the concept of conventional medical materials, and therefore no system has heretofore existed for appropriately evaluating the performance and the like thereof.

It is clear that the number of medical materials prepared as regenerative medicine products will increase in the future, and the Ministry of Health, Labour and Welfare also recommends evaluating quality through animal experiments and the like. According to "Ensuring the Quality and Safety of Cell-Processed Pharmaceuticals, etc. (Yakushokuhatsu No. 0907-3, September 7, 2012, Director-General of the Pharmaceutical and Food Safety Bureau)," animal experiments are recommended as "tests supporting efficacy or performance."

In summary, ordinarily, autotransplantation refers to an Auto-graft, and the transplant material (graft) is transplanted, from collection, 1. is transplanted promptly without undergoing steps such as processing, into a tissue equivalent to the original tissue, 2. is transplanted in the collected state, and 3. is used with the original properties/traits unchanged. Blood is transfused into blood vessels, liver transplantation is performed into the liver, and skin is transplanted as a skin graft.

The autotransplantation referred to in the present invention includes "Auto-plast" (a coined term in the present invention), which expands the concept of transplantation, and includes using as a transplant material something manufactured by subjecting self-derived cells and tissues to various processings and, in some cases, as a tissue in a state different from the original traits. Representative examples include regenerative medicine products defined in the three regenerative medicine laws, and the present invention proposes a method broadly enabling design methods and quality evaluation for medical materials using biologically derived materials.

The conventional concept of transplantation is "transplant material (including allogeneic graft)" which subsumes "Auto (autologous)-graft)", and in principle a graft is used (transplanted) in the state in which it was collected. A transplant material that has been collected and subjected to processing is not included in the concept of "graft." Furthermore, there is still no explicit concept of a "plastic graft" created by subjecting a biologically derived material to some processing. The name "Auto-plast" for a transplant material prepared by processing an autologous biologically derived material as a material is a coined term according to the present invention, and the concept includes Auto-plast as a "composite material" combined with artificial materials and the like, and at present no specific verification method for using them in regenerative medicine has been proposed.

In addition, not only tubular structures and membranous structures but also parenchymal organs are included in Auto-plast, and many composite medical materials are included, such as blood-related dosage forms that do not need to have the form of an organ (non-solid organs) and endocrine cells (atypical organs).

Furthermore, as methods for preparing "Auto-plast," it is assumed that many are in vitro (extracorporeal preparation), but some are also assumed to be possible ex vitro/in vivo (intracorporeal preparation), and quality evaluation as a material for transplantation is possible in the present invention regardless of which preparation method is used.

In order to solve the above problems, the present inventors used an inbred mouse expressing EGFP as a donor and subcutaneously administered several times to neonatal wild-type recipients a crude extract containing crude protein extracted from the donor tissue to induce immune tolerance. Thereafter, an artificial transplant scaffold was transplanted into the donor. As a result, the donor tissue and cells adhered to the transplanted artificial transplant scaffold. When a transplant material including the transplant scaffold prepared in the donor body was transplanted into an immunotolerant recipient, immune reactions against donor-derived cells/tissues could be avoided. In addition, engraftment of donor-derived cells and tissues together with the transplant scaffold could be easily observed in the recipient after transplantation using fluorescence observation. The present invention was completed on the basis of these findings.

That is, the present invention provides a method for producing a transplantation model animal for evaluating a transplant material, the method comprising:
obtaining a recipient that is immunotolerant to a donor expressing a marker by administering a crude extract of the donor to a neonatal recipient; and
transplanting, into the immunotolerant recipient, a transplant material including an artificial transplant scaffold that is coated with, adhered to, infiltrated with, or containing tissue or cells of the donor expressing a marker, wherein
the donor and the recipient are inbred animals.

The present invention also provides the method for producing a transplantation model animal for evaluating a transplant material, wherein the transplant material is a tubular structure, a membranous structure, or a parenchymal artificial organ.

The present invention also provides the method for producing a transplantation model animal for evaluating a transplant material, wherein the transplant material is one recovered after being transplanted into the donor body and left in place for 2 to 30 days.

The present invention also provides the method for producing a transplantation model animal for evaluating a transplant material, wherein the crude extract is a crude protein solution extracted from cells or tissue of the donor.

The present invention also provides the method for producing a transplantation model animal for evaluating a transplant material, wherein the marker is a fluorescent protein.

The present invention also provides a method for evaluating the behavior of a transplant material, wherein, in the step of obtaining the recipient that is immunotolerant to the donor, the crude extract is administered to the neonatal recipient by subcutaneous administration, intramuscular administration, intravenous administration, intradermal administration, transdermal administration, or oral administration.

The present invention also provides a method for evaluating the behavior of a transplant material, the method comprising:
producing a transplantation model animal by a production method comprising:
   obtaining a recipient that is immunotolerant to a donor expressing a marker by administering a crude extract of the donor to a neonatal recipient; and
   transplanting, into the immunotolerant recipient, a transplant material including an artificial transplant scaffold that is coated with, adhered to, infiltrated with, or containing tissue or cells of the donor expressing a marker, wherein
   the donor and the recipient are inbred animals; and
observing the marker in the transplantation model animal,
whereby the behavior of the transplant material in the body of the transplantation model animal is evaluated by the marker.

The present invention also provides the transplantation model animal, which is a mouse.

### [Effects of the Invention]

By using the present invention, it is possible to provide a method for evaluating a transplant material that can realize a highly reliable and highly reproducible transplantation experimental system as an alternative to autotransplantation.

### [Brief Description of the Drawings]

[Figure 1] A diagram showing an outline of the test conducted in an example of the present invention.
[Figure 2] A photograph showing a scene in which the donor green mouse was observed by fluorescence observation.
[Figure 3] A photograph showing a recipient mouse subjected to immune tolerance treatment.
[Figure 4] A photograph showing a scene in which a scaffold was transplanted into the donor green mouse; the upper left of Figure 4 shows the transplanted tubular structure, the lower left of Figure 4 shows a scene in which the transplant scaffold is transplanted into the green mouse, the upper right of Figure 4 shows a scene one week after transplantation of the transplant scaffold, and the lower right of Figure 4 shows a scene in which a transplant material including the transplant scaffold recovered from the green mouse was observed by fluorescence observation.
[Figure 5] A photograph showing a scene in which the tubular structure as a transplant scaffold was transplanted into the recipient mouse.
[Figure 6] A photograph showing the transplant material one month after transplantation into the recipient, wherein the left side of Figure 6 shows the tubular structure (transplant scaffold) one month after transplantation, and the right side of Figure 6 shows a scene in which the tubular structure (transplant material) one month after transplantation was observed by fluorescence observation.

### [Description of Embodiments]

The present invention makes it possible to design transplant materials and to verify the performance and quality thereof by providing a transplantation model animal approximating autotransplantation for use in a method for evaluating a transplant material, and a method for evaluating a transplant material.

### (Method for producing a transplantation model animal for evaluating a transplant material)

The transplantation model animal produced by the method for producing a transplantation model animal for evaluating a transplant material of the present invention is a transplantation model animal into which a transplant material has been transplanted and in which immune tolerance has been induced at the time of transplantation. that is, the transplantation model animal produced by the method for producing a transplantation model animal for evaluating a transplant material of the present invention is a recipient that is immunotolerant to the donor and into which a transplant material has been transplanted. Thetransplantation model animal produced by the method for producing a transplantation model animal for evaluating a transplant material of the present invention can be suitably used in an experimental system for observing the biological reaction, course, and the like when a transplant material is transplanted.

The animal used in the method for producing a transplantation model animal for evaluating a transplant material of the present invention is not particularly limited, but may be a non-human animal, for example, a rodent such as a mouse, rat, or guinea pig; a primate such as a monkey or marmoset; a mammal such as a rabbit, ferret, dog, cat, pig, sheep, goat, cow, or horse; a bird; Nematoda such as a nematode; a flatworm such as a planarian; an insect such as a fruit fly; a fish such as a zebrafish; an amphibian such as a newt or frog; or a reptile such as a lizard. The animal is preferably a rodent such as a mouse, rat, or guinea pig, more preferably a mouse.

The animal used in the present invention may be an inbred animal. In the present specification, the term "inbred animal" refers to a genetically uniform population of animals or plants in which there is little or no difference in genetic properties (genetic traits) between generations and between individuals. An inbred animal can be produced by repeating inbreeding (full-sib mating) for about 20 generations or more in order to fix the genotype and excluding individuals having different traits. In an inbred animal, homology of genetic traits at the level of identical twins is ensured among individuals. The inbred animal is not particularly limited, but for example, the Wistar strain can be used in rats, and the DBA/2 strain, C57BL/6 strain, and BALB/c strain can be used in mice. When an inbred animal is used, animals of the same strain can be treated as the same individual.

The method for producing a transplantation model animal for evaluating a transplant material of the present invention comprises a step of obtaining an immunotolerant recipient by administering a crude extract of a donor expressing a marker (hereinafter also simply referred to as a donor) to a neonatal recipient (immune tolerance step), and a step of transplanting, into the immunotolerant recipient, a transplant material including an artificial transplant scaffold that is coated with, adhered to, infiltrated with, or containing tissue or cells of the donor expressing a marker (transplantation step). In the present specification, the phrase that tissue or cells "coat" has the ordinary meaning in the art and includes covering part or all of the transplant scaffold with the tissue or cell. In the present specification, the phrase that tissue or cells "adhere" has the ordinary meaning in the art and includes, for example, attachment of the tissue or cells to part or all of the transplant scaffold, for example, sticking thereto without separating therefrom. In the present specification, the phrase that tissue or cells "infiltrate" has the ordinary meaning in the art and includes, for example, entering into and spreading inside part or all of the transplant scaffold, for example, adhering inside. In the present specification, the phrase that tissue or cells "contain" has the ordinary meaning in the art and includes, for example, covering the whole of the transplant scaffold.

### (Donor and recipient)

In the present specification, the "donor" is an animal that provides tissue or cells contained in the transplant material. In the present specification, the "recipient" is an animal to which the transplant material is transplanted.

### (Immune tolerance step of recipient)

In the immune tolerance step, by administering a crude extract of a donor expressing a marker to a neonatal recipient, immune tolerance to donor tissue is induced in the recipient, thereby obtaining an immunotolerant recipient. In the present specification, "immune tolerance" means a state in which an immunological rejection reaction (immune response) against a specific antigen is suppressed.

The crude extract of the donor (also referred to as a crude extract) is not particularly limited, but may be a crude extract solution of a crushed product derived from tissue or cells collected from the donor, for example, a crude protein solution extracted from tissue or cells. The tissue or cells collected from the donor may include tissue or cells scheduled to be transplanted. More specifically, as the donor-derived crude extract, it is possible to produce a transplantation model animal for evaluating a transplant material of the present invention, and to evaluate the behavior of the transplant material in the body of the transplantation model animal, by using the whole body of a neonatal animal immediately after birth, organs of various parts regardless of whether from an adult animal or a neonatal animal, internal organs such as the brain, subcutaneous fat, limb muscles, liver, lung, heart, and kidney, and supporting tissues including connective tissues around these organs. Among these, for general use, a crude extract obtained by crushing and extracting the whole body of a neonatal animal, which comprehensively contains antigen proteins throughout the whole body, can be used. When more emphasis is placed on nerve tissue, even when a brain-derived crude extract is also blended, it was possible to produce a transplantation model animal for evaluating a transplant material of the present invention and to evaluate the behavior of the transplant material in the body of the transplantation model animal. The formulation of the crude extract may also be changed for each target organ part according to the target organ scheduled to be produced for transplantation.

The crude extract may be prepared as a crude extract solution, for example, by crushing tissue or cells collected from the donor with a homogenizer, then carrying out centrifugation or filtration, and collecting a supernatant containing soluble proteins. The crude protein solution may be one that has been further concentrated or the like. Also, after preparation, one that has been frozen and stored and then thawed may be used. The crude extract can be prepared, briefly, by the following procedure. In the case of the whole body of neonatal animals used for general purposes, the bodies of 3 to 5 animals are preliminarily cut into pieces of several mm in diameter with scissors or the like. Then, physiological saline at ice temperature is added in an amount equal to the weight of the cut tissue. Then, while being maintained on ice at ice temperature (0 to 4°C), the mixture is intermittently crushed with a homogenizer (tissue crushing apparatus) for about 3 to 5 minutes while observing the state of crushing, until a sufficiently concentrated supernatant is extracted, so that the temperature does not rise. Then, the supernatant is obtained by centrifugation. The crushing conditions, such as the rotation speed for centrifugation, may vary depending on the type of the equipment used and the type of tissue, and may be 200 to 1000 revolutions per minute. Also, in order to facilitate temperature control of the tissue to be crushed, crushing may be performed by first freezing the crushing container together with the tissue at -20°C or lower and then adding physiological saline at ice temperature. When a neonatal animal is used as a material, the animal may be frozen and stored at -20°C or lower while placed directly in a tube or similar container, thereby suppressing degradation of antigen proteins caused by tissue destruction. At the time of use, it may be cut immediately before extraction without thawing as it is, and crushed after pouring physiological saline at ice temperature thereon. All of the above procedures are effective for minimizing denaturation and decomposition of antigen proteins due to a rise in temperature. Since an inbred animal species is used as the donor (antigen-presenting animal), tissues derived from a plurality of individuals may be collectively prepared as a crude extract, aliquoted, frozen and stored, and distributed for use in multiple antigen presentations. Also, since it is not necessary to consider individual differences even among neonatal animals, surplus neonatal animals produced irregularly and in non-uniform numbers by self-breeding can also be secured by freezing and storing them as individuals for collecting presented antigens. When a sufficient amount of supernatant cannot be collected, collection may also be performed by wrapping the material in a filter having a pore diameter of 20 µm or less, crushing it under pressure, and squeezing out the extract solution. Live cells must never be used as the protein for antigen presentation. This is because, although they serve as antigen presentation, they may engraft as live cells, and if surviving cells are present, after transplantation experiments are carried out, it becomes impossible to distinguish between cells that have already been engrafted originally and cells that have newly come to exist in the body by transplantation. The antigen-presenting protein must be completely decomposed and absorbed after presentation and once disappear. For that purpose as well, the step of crushing after freezing is effective.

The crude extract to be administered is not particularly limited, but may be in an amount of 1/30 to 1/15 of the body weight of the neonatal recipient containing protein.

The step of administering the donor crude extract to the neonatal recipient is carried out prior to the transplantation step. The method of administering the donor crude extract to the neonatal recipient is not particularly limited, but for example, a method may be used that can induce immune tolerance by causing the donor crude extract incorporated into the recipient to be recognized by antigen-presenting cells, thereby activating T cells and inducing regulatory T cells (Treg) or causing an anergic state. The anergic state is a state in which, as with self-antigens, T cells also become dysfunctional against donor antigens, are not activated, and the immune response is suppressed. Such methods include, for example, subcutaneous administration, intramuscular administration, intravenous administration, intradermal administration, transdermal administration, and oral administration. Further, the method of administering the donor crude extract to the neonatal recipient used in the present invention is not limited to the above-described methods, but may be, for example, local administration, transmucosal administration, intranasal administration, intratracheal administration, sublingual administration, nasal administration, buccal administration, rectal administration, intravaginal administration, intra-arterial administration, intracardiac administration, intraosseous administration, intraperitoneal administration, intraorbital administration, intravitreal administration, subconjunctival administration, suprachoroidal administration, subretinal administration, intra-articular administration, periarticular administration, administration onto the skin, and inhalation administration.

The donor crude extract may be administered to the neonatal recipient once or several times. For example, the donor crude extract may be administered to the neonatal recipient a total of 3 to 5 times every 1 to 2 days. The first administration to the neonatal recipient may be within 3 days after birth, preferably within 24 hours after birth. All administrations to the neonatal recipient may be carried out within 10 days after birth, preferably within 7 days after birth. Also, the donor crude extract is administered to the recipient in an amount capable of causing immune tolerance in the recipient. For example, in the case of a crude extract containing protein in an amount of 1/20 of the body weight of the neonatal recipient, an immunotolerant recipient to the donor can be obtained by carrying out a first administration, within 24 hours after birth, of a donor crude extract in an amount corresponding to about 5% of the neonatal body weight, and then subcutaneously administering, into the abdomen or the like, a donor-derived crude extract in an amount corresponding to about 5% of the neonatal body weight a total of 4 times every 2 days during 7 days after birth.

### (Transplantation step)

In the transplantation step, a transplant material comprising an artificial transplant scaffold that is coated with, adhered to, infiltrated with, or containing tissue or cells of the donor is transplanted into the recipient.

A transplant material including an artificial transplant scaffold that is coated with, adhered to, infiltrated with, or containing tissue or cells of a donor expressing a marker contains, together with the transplant scaffold, a material derived from tissue or cells of the donor. For example, the transplant material may be a transplant scaffold containing tissue or cells of the donor. In the transplant material, the donor tissue or cells may coat the transplant scaffold, or may adhere to, infiltrate, or be contained in the transplant scaffold.

The transplant scaffold is not particularly limited, but may be, for example, a material exhibiting bioabsorbability or a biocompatible material. Materials that can be used for the transplant scaffold include, for example, PLLA: poly-L-lactic acid, PEG: polyethylene glycol, PGA: polyglycolic acid, a copolymer of PGA and PLA, polyhydroxybutyric acid, polycaprolactone, polyethylene succinate, polydioxanone, polybutylene succinate, and the like. It also includes materials based on soluble cellulose such as oxidized cellulose, proteins, peptides, polyamino acids, polysaccharides, polyesters, polyamides, derivatives, crosslinked products, and copolymers thereof, metallic magnesium, calcium carbonate, calcium phosphate, hyaluronic acid, hydroxyapatite, acrylic resins, fluororesins, polyolefins, silicone, polystyrene, polyester, polyurethane, polycarbonate, polyimide, derivatives, crosslinked products, and copolymers thereof, and titanium, silica, zirconia, and the like. The proteins, peptides, and polyamino acids include, for example, collagen, gelatin, α-polylysine, ε-polylysine, polyglutamic acid, polyaspartic acid, fibrin, and fibroin. The polysaccharides include, for example, chitin and chitosan. The polyesters include, for example, polylactic acid, polycaprolactam, polydioxanone, polyglycolic acid, and polyhydroxybutyric acid. The material of the transplant scaffold may be one of the above-described materials, two or more thereof, or a composite of two or more materials.

The transplant scaffold may also be coated with a coating material. The coating material includes, for example, materials based on fibrin, materials based on collagen, materials based on hyaluronic acid, materials based on glycoproteins, and materials based on soluble cellulose such as oxidized cellulose.

The transplant scaffold is not particularly limited, but may be, for example, a tubular structure, a membranous structure, another structure of any shape, or an artificial organ. The tubular structure may be used, for example, as a transplant material for regenerative medicine for a blood vessel, respiratory organ, digestive organ, urinary organ, or the like. The membranous structure may be used, for example, as a transplant material for regenerative medicine for dura mater, peritoneum, fascia, periosteum, synovial membrane, or the like.

The artificial transplant scaffold that is coated with, adhered to, infiltrated with, or containing tissue or cells of the donor may be provided in any form. For example, a transplant material may be formed by seeding donor cells onto the transplant scaffold and culturing the same.

The transplant material may also be one recovered after a transplant scaffold has been transplanted in advance into the body of the donor and left in place for 2 to 30 days. The transplant material may be one obtained by carrying out transplantation into the donor and recovery multiple times. By transplanting the transplant scaffold into the donor and leaving it in place for a certain period, it is possible to cause the donor tissue or cells to adhere sufficiently to, or infiltrate into, the transplant scaffold.

The production method of the present invention may further comprise a step of transplanting the transplant scaffold into the body of the donor, leaving it in place for 2 to 30 days, and then recovering, from the body of the donor, a transplant material including the artificial transplant scaffold that is coated with, adhered to, infiltrated with, or containing tissue or cells of the donor. This step may be carried out at any timing as long as it is carried out at least before the step of transplanting the transplant material into the recipient.

In a transplant material including an artificial transplant scaffold that is coated with, adhered to, infiltrated with, or containing tissue or cells of the donor, the transplant scaffold may be coated with, adhered to, infiltrated with, or contain the donor tissue or cells over the whole thereof, alternatively a part thereof may be coated with, adhered to, infiltrated with, or contain the donor tissue or cell.

The tissue or cells of the donor expressing a marker contained in the transplant material may be the donor tissue or cells itself, or a processed form thereof. The tissue or cells collected from the donor may be separated, cultured, differentiated, selected, proliferated, or cryopreserved as necessary in order to form the transplant material, and may later be thawed. It may also be tissue or cells that has been made into a cell line by subculture.

Cells and various physiologically active substances (such as cytokines and cell growth factors) may also be added to the transplant material by co-culture, spraying, coating, infiltration, or the like. The physiologically active substances include, for example, vascular endothelial growth factor, platelet-derived growth factor, epidermal growth factor, fibroblast growth factor, hepatocyte growth factor, insulin-like growth factor, brain-derived neurotrophic factor, growth differentiation factor, erythropoietin (EPO), transforming growth factor, and bone morphogenetic protein.

The transplant material is not particularly limited, but may be used, for example, as a blood vessel, respiratory organ, digestive organ, urinary organ, dura mater, peritoneum, fascia, synovial membrane, periosteum, or serous membrane.

The transplant material is not particularly limited, but may be transplanted, for example, into an internal organ such as the brain, liver, kidney, or heart of the recipient, or subcutaneously or into a blood vessel of the recipient. The method of transplanting the transplant material into the recipient is not particularly limited, but a conventionally known method may be used.

The tissue or cells of the donor contained in the transplant material is not particularly limited, but may be collected from an adult donor. When the donor is an inbred animal, not only may the very individual from which the crude extract to be administered to the neonatal recipient was prepared be used as the donor, but also another individual of the same strain may be used as the donor, and a transplant material derived from another individual of the same strain may be transplanted into the recipient.

The tissue or cells of the donor may be selected, for example, from the group consisting of fibroblasts, chondroblasts, osteoblasts, angioblasts, myoblasts, epithelial cells, smooth muscle cells, endothelial cells, vascular endothelial cells, fibrous cells, hepatocytes, chondrocytes, epithelial cells, urothelial cells, smooth muscle cells, keratin-producing cells, β cells, small intestinal epithelial cells, epidermal keratinocytes, bone marrow mesenchymal cells, cardiomyocytes, intervertebral disc cells, gastrointestinal mucosal epithelial cells, ureteral epithelial cells, skeletal articular synovial cells, periosteal cells, perichondrial cells, skeletal muscle cells, smooth muscle cells, cardiomyocytes, pericardial cells, dural cells, meningeal cells, pericytes, glial cells, nerve cells, amniotic cells, placental membrane cells, serosal cells, ependymal cells, periventricular area cells, oral mucosal epithelial cells, nerve cells, and dendritic cells, genetically modified cells thereof, cells produced by gene manipulation techniques such as genome editing or by transdifferentiation techniques using mRNA (messenger RNA) or the like, and cells obtained by culturing and/or differentiating these cells.

The tissue or cells of the donor may also be selected, for example, from the group consisting of somatic tissue stem cells, somatic tissue progenitor cells, germline stem cells, umbilical cord blood stem cells and differentiated cells thereof, adipose stem cells, neural stem cells, neural progenitor cells, dental pulp stem cells, mesenchymal stem cells, blood stem cells, hematopoietic stem cells, oral mucosal stem cells, periodontal ligament stem cells, hepatic stem cells, bone marrow mesenchymal stem cells, undifferentiated stem cells, undifferentiated progenitor cells, predifferentiated stem cells, predifferentiated progenitor cells, keratin-producing cell progenitor cells, organ-specific stem cells, progenitor cells of organ-specific stem cells, genetically modified cells thereof, and genome-edited cells. The somatic tissue stem cells include, as human somatic stem cells, those defined in the Notice of the Director-General of the Pharmaceutical and Food Safety Bureau, Ministry of Health, Labour and Welfare, dated September 7, 2012, Yakushokuhatsu No. 0907-3, "Ensuring the Quality and Safety of Human Allogeneic Somatic Stem Cell-Processed Pharmaceuticals, etc." "Human somatic stem cell" means a cell collected from a human or a cell produced by division of the cell, which has multipotency and maintains self-renewal ability or is presumed to be similar thereto, and among cells derived therefrom, refers to the following: tissue stem cells (for example, hematopoietic stem cells, neural stem cells, mesenchymal stem cells (including bone marrow stromal stem cells and adipose tissue-derived stem cells), corneal epithelial stem cells, skin stem cells, hair follicle stem cells, intestinal stem cells, hepatic stem cells, and skeletal muscle stem cells) and cell populations rich therein (for example, total bone marrow cells including hematopoietic stem cells). Human somatic stem cells include vascular progenitor cells, umbilical cord blood, and bone marrow stromal cells. Human somatic stem cells also include cells obtained by culturing and/or differentiating these cells ex vivo.

The tissue or cells of the donor may also be "artificial pluripotent stem cells" and "artificial pluripotent stem cell-like cells," such as iPS cells, produced by gene manipulation techniques such as genetic recombination and genome editing and by transdifferentiation techniques using mRNA (messenger RNA) or the like, as well as cells obtained by culturing and/or differentiating these cells.

The tissue or cells of the donor may be, for example, an adipose stem cell collected from subcutaneous fat, a neural stem cell collected from the brain, a bone marrow-derived stem cell, a dental pulp stem cell collected from a tooth, a mesenchymal stem cell collected from oral submucosal tissue, or an immune cell contained in the thymus, bone marrow, lymph node, or the like.

### (Method for producing a transplantation model animal for evaluating a transplant material using a donor expressing a marker)

A method for producing a transplantation model animal for evaluating a transplant material using a donor expressing a marker of the present invention comprises a step of obtaining a recipient that is immunotolerant to a donor expressing a marker by administering a crude extract of the donor to a neonatal recipient, and a step of transplanting, into the immunotolerant recipient, a transplant material including an artificial transplant scaffold that is coated with, adhered to, infiltrated with, or containing tissue or cells of the donor expressing a marker.

In the present specification, "marker" means something that makes it possible to distinguish and observe cells expressing the marker from cells not expressing the marker. The marker may be any marker that enables identification of donor-derived cells after transplantation into the recipient. The marker may be, for example, a fluorescent protein. Examples of the fluorescent protein include green fluorescent protein (GFP) derived from Aequorea victoria; variants of GFP such as EGFP; GFP mutants emitting fluorescence of different colors (for example, blue fluorescent protein (BFP), yellow fluorescent protein (YFP), and cyan fluorescent protein (CFP)); dsRed fluorescent protein (dsRed2FP); red fluorescent protein (eqFP611) isolated from Entacmaea quadricolor; cyan fluorescent protein (amFP486 or AmCyan1) isolated from Anemonia majano; fluorescent protein (Azami Green) isolated from Galaxeidae; fluorescent protein (ZSGREEN) isolated from Zoanthus; and any other fluorescent protein.

When the marker is a fluorescent protein such as EGFP, it can be constantly and stably expressed in vivo, and donor-derived cells can be easily observed by fluorescence in the body of the recipient after transplantation. Further, when the marker is a fluorescent protein such as EGFP, since the fluorescence is due to excitation light irradiation, it is not necessary to subject the tissue to staining treatment or the like for observation, and the observed tissue can be easily observed while remaining alive. When the marker is EGFP, for example, by using excitation light of 470 nm to 530 nm, particularly excitation light of 488 nm, which is specific excitation light for EGFP, green fluorescence having a maximum fluorescence wavelength of 507 nm can be obtained.

A donor expressing a marker is an animal into which a marker gene has been introduced and in which the marker is constitutively and stably expressed (or can be expressed) in some or all cells in the body. A donor expressing a marker can be produced by introducing a marker gene by a conventionally known method.

In the method of the present invention, the recipient is not particularly limited, but may be an animal into which at least the marker introduced into the donor has not been introduced, for example, a wild-type animal (wild-type mouse: WT) that has not been genetically manipulated. Further, it may also be an inbred animal in order to improve experimental accuracy.

Since the recipient has not been introduced with the marker, when transplantation is carried out by an ordinary transplantation method, there is a high possibility that an immunological rejection reaction will be induced by the marker contained in donor-derived cells and other donor-derived antigens. However, according to the method of the present invention, by administering a crude extract of a donor expressing a marker to the neonatal recipient in advance, immune tolerance can be induced to donor-derived antigens including the marker, and therefore the immune reaction at the time of transplantation can be suppressed only with respect to donor-derived antigens. On the other hand, with respect to antigens not derived from the donor, induction of a normal immune reaction is maintained.

By using a donor expressing a marker, donor-derived cells and tissues can be easily observed in the recipient after transplantation. Further, cells, tissues, and the like generated in the recipient after transplantation can be easily distinguished as being recipient-derived or donor-derived. Therefore, for example, when a tumor or the like occurs in the recipient after transplantation, it can be easily determined whether it has arisen from donor-derived cells or from recipient-derived cells.

### (Method for evaluating the behavior of a transplant material)

A method for evaluating the behavior of a transplant material of the present invention first comprises a step of producing a transplantation model animal by a production method for a transplantation model animal approximating autotransplantation; wherein the production method comprises a step of obtaining a recipient that is immunotolerant to a donor expressing a marker by administering a crude extract of the donor to a neonatal recipient, and a step of transplanting, into the immunotolerant recipient, a transplant material including an an artificial transplant scaffold that is coated with, adhered to, infiltrated with, or containing tissue or cells of the donor expressing a marker. The method for producing the transplantation model animal as the transplantation model animal is as described above in "Method for producing a transplantation model animal for evaluating a transplant material." The donor and the recipient are inbred animals. Further, the transplantation model animal produced by the method for producing a transplantation model animal for evaluating a transplant material of the present invention is a transplantation model animal into which a transplant material has been transplanted and in which immune tolerance has been induced at the time of transplantation.

The method for evaluating the behavior of a transplant material of the present invention further comprises a step of observing the transplant material in the recipient into which the transplant material has been transplanted. Thereby, the behavior of the transplant material in the body of the transplantation model animal can be evaluated. In the present specification, "evaluate" includes observing. Further, evaluating includes evaluating whether the transplant scaffold used in the transplant material has quality suitable for transplantation. Quality suitable for transplantation includes, for example, biocompatibility, functionality at the transplanted site, and the presence or absence of toxicity. Evaluating includes, for example, evaluating the presence or absence of biocompatibility of the transplant material by observing the immune reaction and local reaction of the immunotolerant recipient after transplantation of the transplant material. Evaluating also includes evaluating functionality by confirming the function of the transplanted site. Evaluating also includes evaluating the presence or absence of toxicity by observing the general condition of the recipient, observing changes in body weight, appetite, and behavior, and performing blood tests, urine tests, and the like.

Evaluating also includes confirming whether donor-derived new blood vessels are formed around the transplant scaffold or whether donor-derived cells are engrafted, and, when these are observed, evaluating that the transplant scaffold has biocompatibility. Since the method for evaluating the behavior of a transplant material of the present invention uses a donor expressing a marker, donor-derived cells and recipient-derived cells can be easily distinguished by using the marker. Thereby, the behavior of the transplant material in the body of the transplantation model animal can be evaluated. Accordingly, in the present specification, "observe" includes observing donor-derived cells, that is, the behavior of the marker, in the transplantation model animal that is the recipient.

### (Transplantation model animal and method for producing the same)

The present invention also provides a transplantation model animal approximating autotransplantation, produced by a method for producing a transplantation model animal, the method comprising:
a step of obtaining a recipient that is immunotolerant to a donor expressing a marker by administering a crude extract of the donor to a neonatal recipient; and
a step of transplanting, into the immunotolerant recipient, a transplant material including an an artificial transplant scaffold that is coated with, adhered to, infiltrated with, or containing tissue or cells of the donor expressing a marker.

The present invention also provides a method for producing a transplantation model animal approximating autotransplantation, the method comprising a step of obtaining a recipient that is immunotolerant to a donor expressing a marker by administering a crude extract of the donor to a neonatal recipient, and a step of transplanting, into the immunotolerant recipient, a transplant material including an an artificial transplant scaffold that is coated with, adhered to, infiltrated with, or containing tissue or cells of the donor expressing a marker. The transplantation model animal of the present invention can be used for evaluating a transplant material approximating autotransplantation.

The transplantation model animal of the present invention can be produced by a method comprising the immune tolerance step and the transplantation step described above in "Method for producing a transplantation model animal for evaluating a transplant material using a donor expressing a marker." That is, the transplantation model animal of the present invention is produced by obtaining a recipient that is immunotolerant to a donor expressing a marker by administering a crude extract of the donor to a neonatal recipient, and then transplanting, into the immunotolerant recipient, a transplant material including an an artificial transplant scaffold that is coated with, adhered to, infiltrated with, or containing tissue or cells of the donor expressing a marker. Details of the immune tolerance step and the transplantation step are as described above in "Method for producing a transplantation model animal for evaluating a transplant material."

In the transplantation model animal produced as described above, the transplanted transplant material is labeled with a marker. Accordingly, in the transplantation model animal of the present invention, it is possible to easily confirm not only the behavior of the transplantation model animal as the recipient, but also the behavior of the transplanted transplant material in the body of the recipient transplantation model animal. It is possible to easily observe by fluorescence where donor-derived cells and the like are present in the recipient.

In the transplantation model animal of the present invention, since the transplant material is transplanted after immune tolerance to the donor has been induced in the recipient in advance, the immune reaction of the transplantation model animal as the recipient to the transplant material is suppressed. Accordingly, for example, it can be used in an experimental system for observing the biological reaction, course, and the like when a transplant material is transplanted. On the other hand, immune reactions to all antigens that are not donor-derived are maintained, and therefore, with respect to 1.(<1>) the risk of interruption of the experiment due to infection during the course, and 2.(<2>) the risk of an increase in carcinogenic risk, and the like, it is possible to expect experimental results closer to clinical transplantation than ever before.

By using an inbred animal, the present invention can provide a so-called "animal experimental model approximating autotransplantation." By using an inbred animal, the present invention can easily induce immune tolerance even in an animal having normal immune function, and can establish an animal model capable of avoiding an immune reaction to a specific individual. Therefore, according to the present invention, it is not necessary to use a conventional immunodeficient animal experimental system, and transplantation experiments can be carried out under conditions close to clinical practice using an animal having normal immune function. Further, according to the present invention, small animals such as mice, in which autotransplantation has originally been difficult, can be used in an experimental system for observing the biological reaction, course, and the like when a transplant material is autotransplanted. Accordingly, it is possible to provide an alternative to autotransplantation experiments that could be carried out only in medium-sized or large animals, and, while also conforming to trends in animal welfare, it is also possible to comply with the recommendations of the Ministry of Health, Labour and Welfare relating to development of regenerative medicine products under relevant laws and regulations related to regenerative medicine.

In the future, various regenerative medicine products will be researched, developed, and commercialized, and most of them will be artificial organs that has never been obtained by humans. By using the transplantation model animal of the present invention, with respect to artificial organs for which there is no prior performance information, various conditions can be examined in advance by animal experiments and the like before providing them for human clinical practice, and designs, performance, and surgical procedures more suitable for clinical practice can be fully examined in advance.

Further, by using the transplantation model animal of the present invention, it is possible, by immune tolerance, to carry out experiments under conditions even closer to actual clinical medicine, as compared with conventionally performed animal experiments having the same purpose (such as experimental systems using immunodeficient animals).

Heretofore, transplantation therapy has been based on "homologous use" of the organ of origin. Since the function (performance) of the transplanted organ/tissue had been confirmed and ensured in advance in the body of the donor individual, it was unnecessary, and should not have been carried out, to evaluate the performance of the organ alone prior to transplantation. In contrast, although regenerative medicine products can be evaluated for performance ex vivo, their in vivo performance is not ensured in advance, and therefore, in principle, functional evaluation in vivo after transplantation should be carried out in advance by animal experiments or the like. Also, in conventional transplantation, the quality of function after transplantation depends exclusively on the success or failure of the operation at the time of transplantation and suppression of immune rejection. In this respect, conditions suitable for transplantation operation should also be studied in advance for regenerative medicine products, and in addition, production of products that can avoid immune rejection and the like as much as possible should be studied.

By using the transplantation model animal of the present invention, it becomes possible to carry out experiments approximating autotransplantation, which have conventionally been impossible in small animals, and therefore it becomes possible to design and develop regenerative medicine products using "autologous biological tissue" as a material, as organs to be embedded semi-permanently in the body as living tissue. Thereby, it is possible to produce a transplant material that does not require measures against immune reaction. That is, this leads to development of artificial organs that does not cause risks such as prolongation of an immunosuppressed state due to permanent use of immunosuppressants (infection, carcinogenicity), and in addition makes it possible to reduce permanent treatment costs (such as immunosuppressants). In view of these points, in regenerative medicine, the most important thing is to ensure better performance and quality as a medical material for transplantation including regenerative medicine products. Furthermore, by using the transplantation model animal of the present invention, it also becomes possible to greatly reduce the necessary expenses involved in carrying out experiments (initial costs and continuing costs), because widely used inexpensive materials can be selected for experimental materials (experimental animals such as mice and materials for raising them) and research facilities (clean rooms and facilities for preventing diffusion of genetically modified animals). Immunodeficient animals for experiments are expensive, require special facilities and special feed, have high maintenance costs, and are also susceptible to infection. The transplantation model animal of the present invention has normal immune function, and immune responses to non-donor antigens remain intact, and therefore the initial cost and maintenance cost are also inexpensive, the model is resistant to infection, and there are few disadvantages in breeding.

Also, by using the transplantation model animal of the present invention, it is unnecessary to use medium-sized or large animals, which have been indispensable for conventional autotransplantation experimental systems, and therefore there are advantages also in terms of cost and labor without conflicting with social trends such as animal welfare. As a result, since it is easy to secure a large number of experimental cases and the lifespan is short, it is also possible to deal with different biological conditions in each life stage, thereby making it possible to design and evaluate the performance of various transplant medical materials under different conditions. For example, when life stages are divided into childhood (pre-reproductive period), reproductive period, and post-reproductive period (senescent period), in experiments on senescent small animals, an appropriate age stage is reached after rearing for several months. During that time, even when time is required for processing donor-derived transplant candidate cells (cell processing such as conversion into iPS cells and various differentiation treatments, and production of composite transplant materials with autologously organized materials and bioabsorbable materials), if immune tolerance treatment has been carried out using inbred animal species also for the donor, multiple individuals at each life stage can be simultaneously subjected to experiments. As a result, since multiple experiments can be carried out at the same time, it is possible not only to evaluate the performance of the produced artificial organ itself, but also to evaluate the effects of variations in surgical procedures and differences in concomitant drugs and devices. Eventually, in accordance with differences in surgical conditions and recipient-specific conditions, the design of more customized artificial organs (= regenerative medicine products) can also be added as a subject of consideration.

### [Examples]

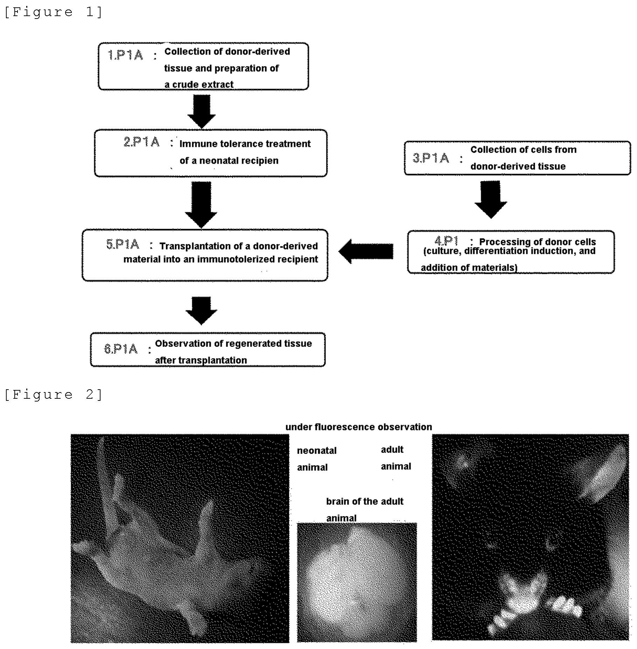

Figure 1 shows an outline of the test conducted in the Examples. The abbreviations used in the following Examples are also explained below.

### Explanation of terms:

GM: Green Mouse (an inbred genetically modified mouse constitutively expressing EGFP),
ICR mouse: an outbred experimental albino mouse strain that is highly prolific and the most popular.
P1, P1A: experimental facilities compliant with specific measures (containment measures) for preventing diffusion of genetically modified organisms in compliance with the international treaty "Cartagena Protocol," wherein P1 and P1A are among the least stringent conditions. P1 is a facility required mainly for handling cells, and P1A is a facility required mainly for handling animals.

1. P1A (animal experimental facility): collection of GM-derived tissue and preparation of crude protein
   a step of collecting a donor-derived antigen-presenting protein for inducing immune tolerance in a recipient
2. P1A (animal experimental facility): immune tolerance treatment of ICR mice (wild-type)
   a step of administering an antigen protein to a neonatal recipient to render the recipient immunotolerant
3. P1A (animal experimental facility)
   a step of collecting cells and tissues to be transplant candidates from a donor
4. P1 (cell experimental facility)
   a step of processing collected donor-derived cells and the like as necessary, or preparing the same as a transplant material
5. P1A (animal experimental facility)
   a step of transplanting, into an immunotolerant recipient, a material collected from a donor
6. P1A (animal experimental facility)
   a step of observing the post-transplantation state at an appropriate time, evaluating the performance of the transplant material, and considering the design of the transplant material as necessary.

### (Donor)

As a donor, a genetically modified inbred mouse expressing EGFP (Enhanced green fluorescence protein: green fluorescent protein derived from Aequorea victoria) throughout the whole body by genetic recombination (Green Mouse: C57BL/6-(CAG-EGFP) C14-Y01-FM131Osb (RBRC00267: RIKEN BioResource Center)) was used (hereinafter also referred to as "Green Mouse (GM)"). The inbred mouse means a mouse obtained by repeated breeding within the same species and having homology of expressed traits corresponding to monozygotic twins between generations and/or between individuals in genetic traits and expression of the traits.

Figure 2 is a photograph showing fluorescence observation of the donor Green Mouse. By irradiating excitation light (ultraviolet light in the case of GM), green fluorescence due to intracellular EGFP can be confirmed. In neonatal animals, fluorescence could be confirmed in the whole-body epidermis (left side of Figure 2), and in adult animals, fluorescence could be confirmed in the excised brain (center of Figure 2) and in exposed epidermis excluding body hair (right side of Figure 2), respectively.

### (Preparation of donor-derived crude extract)

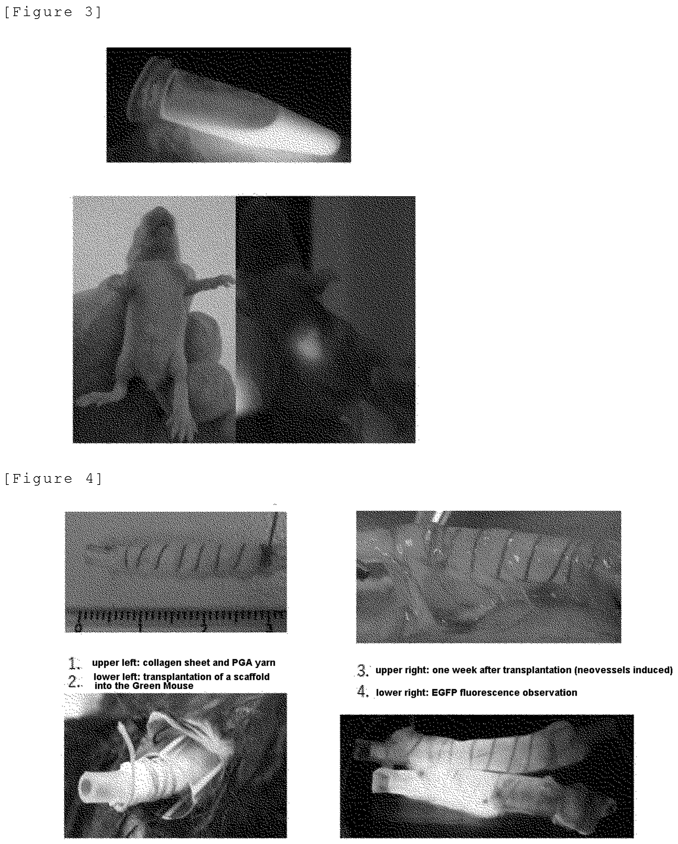

Tissue was collected from the donor Green Mouse, crushed with a homogenizer (tissue crushing apparatus), and then centrifuged or filtered to recover a solution. Specifically, the whole body of 3 to 5 neonatal animals was preliminarily cut into pieces of several mm in diameter with scissors or the like. Then, physiological saline at ice temperature was added in an amount equal to the weight of the cut tissue. Then, while being maintained on ice at ice temperature (0 to 4°C), the tissue was intermittently crushed with a homogenizer (tissue crushing apparatus) for about 3 to 5 minutes while observing the state of crushing, until a sufficiently concentrated supernatant was extracted, so that the temperature does not rise. Then, a supernatant was obtained by centrifugation (200 to 1000 rpm). This solution containing soluble protein was used as the crude extract. The crude extract is also referred to as a crude protein solution (Step 1 in Figure 1). The upper part of Figure 3 shows the recovered crude protein solution. As shown in the figure, when irradiated with EGFP-specific excitation light, it can be confirmed that the crude extract in the test tube has fluorescence. In order to efficiently induce immune tolerance, a crude extract containing a transplant candidate tissue may also be prepared, or the crude extract may be further purified for use.

### (Immune tolerance step)

As the recipient, an ICR mouse (wild-type inbred strain: Wild-type), which is commercially available as an experimental organism, was use. The ICR mouse was subjected to immune tolerance treatment (Step 2 in Figure 1). An initial dose of donor crude extract in an amount of about 5% BW was administered subcutaneously into the abdominal subcutaneous tissue of a neonatal ICR mouse within 24 hours after birth. Thereafter, during 7 days after birth, the donor-derived crude extract was subcutaneously administered into the abdomen a total of 4 times every 2 days. The amount of the crude extract administered per subcutaneous injection corresponded to 1/20 of the body weight of the recipient.Thereafter, the mouse was raised normally for 4 weeks or more.

Figure 3 is a photograph showing the recipient mouse subjected to immune tolerance treatment. The donor-derived crude extract in the test tube showing green fluorescence when irradiated with EGFP-specific excitation light in the upper part of Figure 3 was subcutaneously administered into the abdominal midline of an ICR neonatal mouse (lower left of Figure 3). Then, when the same excitation light was irradiated onto the abdomen, the presence of the green fluorescent protein could be confirmed through the skin (lower right of Figure 3). The donor-derived crude extract incorporated into the recipient is considered to have induced immune tolerance by being recognized by antigen-presenting cells in the body of the recipient, activating T cells, and inducing regulatory T cells (Treg) or causing an anergic state.

### (Preparation of composite material for transplantation)

As the transplant scaffold for transplantation, a tubular structure having a silicone tube as a core was prepared. This tubular structure can be used as a pseudo-blood vessel. Further, this tubular structure can also be used as a membranous structure when unfolded. As a prototype, a tube made of a biocompatible material such as silicone (product name: Safeed Silicone Catheter; outer diameter 2.7 mm) (hereinafter, "silicone tube") was used in the Example. A white collagen sheet, which is a self-organizing material (product name: IntegraN; sheet type 45 × 30 mm) (hereinafter, "collagen sheet"), was wound so as to form two or more layers around the silicone tube. Then, blue-violet bioabsorbable PGA yarn (PGA: polyglycolic acid) was further wound therearound and fixed in order to prevent rewinding and loosening (upper left figure of Figure 4). At this point, the wound tubular collagen sheet is not watertight, and therefore water easily leaks therefrom.

Figure 4 is a photograph showing a scene in which a scaffold was transplanted into the donor Green Mouse. The upper left of Figure 4 shows the tubular structure, which is the transplant scaffold transplanted into the donor. The lower left of Figure 4 shows a scene in which the transplant scaffold is transplanted into the Green Mouse. The upper right of Figure 4 shows a scene one week after the transplant scaffold was transplanted into the Green Mouse. One week after transplantation, inductionof (induction of)new blood vessels was observed. The lower right of Figure 4 shows a scene in which a transplant material including the transplant scaffold excised and recovered from the Green Mouse was observed by fluorescence observation.

### (Step of transplanting the transplant scaffold into the donor)

Figure 4 shows the step of transplanting the above tubular structure (transplant scaffold) into the donor Green Mouse (lower left figure of Figure 4). An approximately 1 cm skin incision was made in the back of an adult Green Mouse serving as the donor, and then the subcutaneous tissue was dissected in a tunnel shape to a length of about 3 cm to prepare an insertion cavity. An outer sheath was inserted into the insertion cavity, and the tubular structure was inserted subcutaneously therethrough. With the whole inserted tubular structure in a state in which it could be embedded subcutaneously without difficulty, the outer sheath was withdrawn and the insertion hole was sutured and closed.

One week after subcutaneous embedding transplantation, the skin of the transplanted Green Mouse was incised and reflected to an extent such that the whole tubular structure could be seen, and the entire tubular structure was confirmed (upper right of Figure 4). The wound collagen sheet was moistened with interstitial fluid and the like, was coated with cells derived from the Green Mouse, and had become semitransparent. It was connected to the surrounding surrounding host tissue by membranous connective tissue. Using this connective tissue as a basis, abundant bridging of new blood vessels was observed in the collagen tube, and it was confirmed that fine blood vessels had extended over the entire circumference.

The tubular structure one week after transplantation was excised and recovered as a transplant material (lower right of Figure 4; corresponding to Steps 3 and 4 in Figure 1). The collagen tube was excised at the connective tissue portion connected to the surrounding host tissue so as not to damage the collagen tube.

When the excised tubular structure was irradiated with excitation light, the transplant material emitted fluorescence, showing that Green Mouse-derived cells had been uniformly seeded throughout the collagen tube. At this stage, it was also separately confirmed that watertightness with no water leakage had already been acquired. Further, by culturing from the excised collagen tube, the cells seeded on the collagen sheet were live cells that were also capable of being cultured.

### (Step of transplanting the transplant material into the immunotolerant recipient)

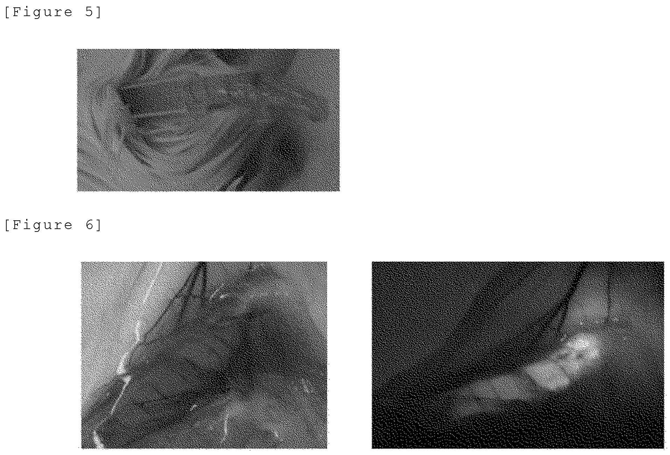

With respect to a wild-type ICR mouse having no marker, in which immune tolerance to the Green Mouse had been induced, an operation similar to that described above in "Preparation of composite material for transplantation" was carried out, and the transplant material including the tubular structure recovered from the Green Mouse was subcutaneously transplanted. Specifically, an approximately 1 cm skin incision was made in the back of the ICR mouse, the subcutaneous tissue was dissected in a tunnel shape to a length of 3 cm, and an insertion cavity was prepared. An outer sheath was inserted into the insertion cavity, and the tubular structure was inserted subcutaneously therethrough (Figure 5). With the whole inserted tubular structure in a state in which it could be embedded subcutaneously without difficulty, the outer sheath was withdrawn and the insertion hole was sutured and closed.

One month after subcutaneous embedding transplantation, the skin was incised and reflected to an extent such that the whole tubular structure could be seen, and the entire tubular structure was confirmed (left side of Figure 6). The wound collagen sheet was moistened with interstitial fluid and the like, and was coated with tissue cells and the like derived from the ICR mouse over the cell layer derived from the Green Mouse, whereby transmission of visible light was reduced. It was firmly connected to the surrounding surrounding host tissue by connective tissue. Using this connective tissue as a basis, abundant bridging of neovessels was observed in the tubular structure, and it was confirmed that new blood vessels derived from the Green Mouse had further developed and that blood vessels had spread over the entire circumference.

Further, since Green Mouse-specific fluorescence was confirmed by irradiation with excitation light (right side of Figure 6), it was confirmed that, even in subcutaneous tissue, in which a particularly strong immune reaction is likely to be induced, Green Mouse-derived cells had engrafted in the body of the immunotolerized wild-type ICR mouse without undergoing immune rejection. Further, from tissue collected from the tubular structure transplanted into the ICR mouse, culturable live cells were confirmed, and coexistence of cells emitting green fluorescence and cells having no fluorescence was confirmed.

Figure 5 is a photograph showing a scene in which a transplant material including the tubular structure was transplanted into the recipient mouse.

Figure 6 is a photograph showing the transplant material one month after transplantation into the recipient. The left side of Figure 6 shows the tubular structure (transplant material) one month after transplantation, and the right side of Figure 6 shows a scene in which the tubular structure (transplant material) one month after transplantation was observed by fluorescence observation. One month after transplantation, fluorescence observation revealed the distribution of donor-derived cells and tissues attached to the tubular structure and new cells and tissues and the like produced therefrom.

### [Industrial Applicability]

The present invention is usable as a highly reliable and highly reproducible transplantation experimental system. It also contributes to evaluation of the design, performance, and surgical procedure of unknown artificial organs that will be developed in the future.

## Claims

1. A method for producing a transplantation model animal for evaluating a transplant material, the method comprising:
obtaining a recipient that is immunotolerant to a donor expressing a marker by administering a crude extract of the donor to a neonatal recipient; and
transplanting, into the immunotolerant recipient, a transplant material including an artificial transplant scaffold that is coated with, adhered to, infiltrated with, or containing tissue or cells of the donor expressing a marker, wherein
the donor and the recipient are inbred animals.

2. The method for producing a transplantation model animal for evaluating a transplant material according to claim 1, wherein the transplant material is a tubular structure, a membranous structure, or an artificial organ.

3. The method for producing a transplantation model animal for evaluating a transplant material according to claim 1 or 2, wherein the transplant material is one recovered after the transplant material was transplanted into the body of the donor and left in place for 2 to 30 days.

4. The method for producing a transplantation model animal for evaluating a transplant material according to claim 1 or 2, wherein the crude extract is a crude protein solution extracted from cells or tissue of the donor.

5. The method for producing a transplantation model animal for evaluating a transplant material according to claim 4, wherein the marker is a fluorescent protein.

6. The method for producing a transplantation model animal for evaluating a transplant material according to claim 1 or 2, wherein, in the step of obtaining the recipient that is immunotolerant to the donor, the crude extract is administered to the neonatal recipient by subcutaneous administration, intramuscular administration, intravenous administration, intradermal administration, transdermal administration, or oral administration.

7. A method for evaluating the behavior of a transplant material, the method comprising:
producing a transplantation model animal by a production method comprising:
obtaining a recipient that is immunotolerant to a donor expressing a marker by administering a crude extract of the donor to a neonatal recipient; and
transplanting, into the immunotolerant recipient, a transplant material including an artificial transplant scaffold coated with, adhered to, infiltrated with, or containing tissue or cells of the donor expressing a marker, wherein
the donor and the recipient are inbred animals; and
observing the marker in the transplantation model animal,
whereby the behavior of the transplant material in the body of the transplantation model animal is evaluated by the marker.
